(19) **Europäisches Patentamt**
**European Patent Office**
**Office européen des brevets**

(11) **EP 1 853 164 B1**

(12) **FASCICULE DE BREVET EUROPEEN**

(45) Date de publication et mention de la délivrance du brevet:
**16.06.2010 Bulletin 2010/24**

(21) Numéro de dépôt: **06709361.7**

(22) Date de dépôt: **22.02.2006**

(51) Int Cl.:
*A61B 5/107* (2006.01)        *G01B 11/03* (2006.01)

(86) Numéro de dépôt international:
**PCT/FR2006/000400**

(87) Numéro de publication internationale:
**WO 2006/092479 (08.09.2006 Gazette 2006/36)**

(54) **INSTALLATION ET PROCEDE DE MESURE D' UNE CARACTERISTIQUE GEOMETRIQUE D UN SEGMENT ANATOMIQUE D UN SUJET ET PROGRAMME D ORDINATEUR METTANT EN OEUVRE UN TEL PROCEDE**

INSTALLATION UND VERFAHREN ZUR MESSUNG EINER GEOMETRISCHEN EIGENSCHAFT EINES ANATOMISCHEN SEGMENTS EINER PERSON UND COMPUTERPROGRAMM ZUR UMSETZUNG DIESES VERFAHRENS

INSTALLATION AND METHOD FOR MEASURING A GEOMETRIC CHARACTERISTIC OF AN ANATOMICAL SEGMENT OF AN INDIVIDUAL AND COMPUTER PROGRAM IMPLEMENTING ONE SUCH METHOD

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **28.02.2005 FR 0502021**

(43) Date de publication de la demande:
**14.11.2007 Bulletin 2007/46**

(73) Titulaires:
- **Di Mascio, Gérard**
  **76420 Bihorel (FR)**
- **Lecerf, Arnaud**
  **76440 Sommery (FR)**
- **Held, Eric**
  **27200 Vernon (FR)**

(72) Inventeurs:
- **Di Mascio, Gérard**
  **76420 Bihorel (FR)**
- **Lecerf, Arnaud**
  **76440 Sommery (FR)**
- **Held, Eric**
  **27200 Vernon (FR)**

(74) Mandataire: **Cabinet Plasseraud**
**52, rue de la Victoire**
**75440 Paris Cedex 09 (FR)**

(56) Documents cités:
**US-A- 5 016 173        US-A- 5 457 325**
**US-A1- 2001 030 754        US-A1- 2004 032 595**
**US-A1- 2004 228 517**

EP 1 853 164 B1

Printed by Jouve, 75001 PARIS (FR)

**Description**

La présente invention est relative aux installations et aux procédés de mesure d'une caractéristique géométrique d'un segment anatomique d'un sujet et aux programmes d'ordinateur mettant en oeuvre de tels procédés.

On veut pouvoir déterminer des caractéristiques géométriques d'un sujet humain dans une position donnée, telles que par exemple les positions respectives de deux points anatomiques, les longueurs respectives ou l'orientation relative de deux segments anatomiques, ou autres.

Dans ce but, il est connu d'utiliser une installation de métrologie, telle que décrite dans DE 4216458. Néanmoins, l'utilisation de matrices de capteurs à placer sur le corps est problématique, en particulier car celles-ci doivent être fixées sur le corps et provoquent en conséquence des contraintes sur la posture du sujet soumis à la mesure.

En outre, on est limité quant au nombre de mesures obtenues, par le nombre et la position des matrices de capteurs utilisées. En particulier, il n'est pas possible de mesurer la position de nombreux points auxquels il n'est pas possible de fixer une matrice de capteurs.

On connaît pas ailleurs des méthodes de mesure dans US-5,016,173, US2004/032595, US2004/228517, US-5,457,325, US2001/030754. Toutefois, les mesures de l'art antérieur sont absolues et ne se réfère pas à un modèle anatomique précis.

La présente invention a notamment pour but de pallier ces inconvénients.

Elle propose à cet effet une installation de mesure conforme à la revendication 1.

Grâce à ces dispositions, on peut mesurer de nombreuses informations géométriques du sujet, sans être limité aux emplacements des matrices de capteur. Il suffit que le segment anatomique soit détectable sur deux des images planes. Quand l'installation est utilisée pour une métrologie de l'être humain, celui-ci peut prendre sa posture naturelle dans l'espace de mesure, ce qui permet d'établir une caractérisation pertinente de sa posture.

Des modes de réalisation avantageux de l'installation sont visés dans les revendications 2 à 10.

L'invention se rapporte aussi à un procédé de mesure selon la revendication 11.

- On peut également prévoir de mettre en oeuvre le procédé selon l'une des revendications dépendantes 12 et 13.

Selon un autre aspect encore, l'invention se rapporte à un programme d'ordinateur comprenant des codes de programme pour la mise en oeuvre de ce procédé de mesure lorsqu'il est exécuté sur une machine programmable.

D'autres caractéristiques et avantages de l'invention apparaîtront au cours de la description suivante d'une de ses formes de réalisation, donnée à titre d'exemple non limitatif, en regard des dessins joints.

Sur les dessins :

- la figure 1 est une vue en perspective d'un mode de réalisation de l'installation,
- la figure 2 est une vue plane de face du dispositif de visée de la figure 1,
- les figures 3a, 3b et 3c sont des schémas explicatifs du principe de calibration de l'installation,
- la figure 4 est un schéma du système informatique,
- la figure 5 est un schéma anatomique artistique expliquant la position d'un certain nombre de points anatomiques du sujet,
- la figure 6 est un schéma plan explicatif de l'obtention de la position du centre des pieds,
- la figure 7 est un schéma explicatif du positionnement de trois points anatomiques au niveau du pied,
- la figure 8 est une vue schématique d'une fenêtre d'un exemple de logiciel de supervision utilisé conjointement avec l'installation, et
- la figure 9 est une vue d'écran comprenant trois fenêtres illustrant les positions des vertèbres respectivement en vue de dessus, de face et de profil, pour un sujet en posture naturelle.

Sur les différentes figures, les mêmes références désignent des éléments identiques ou similaires.

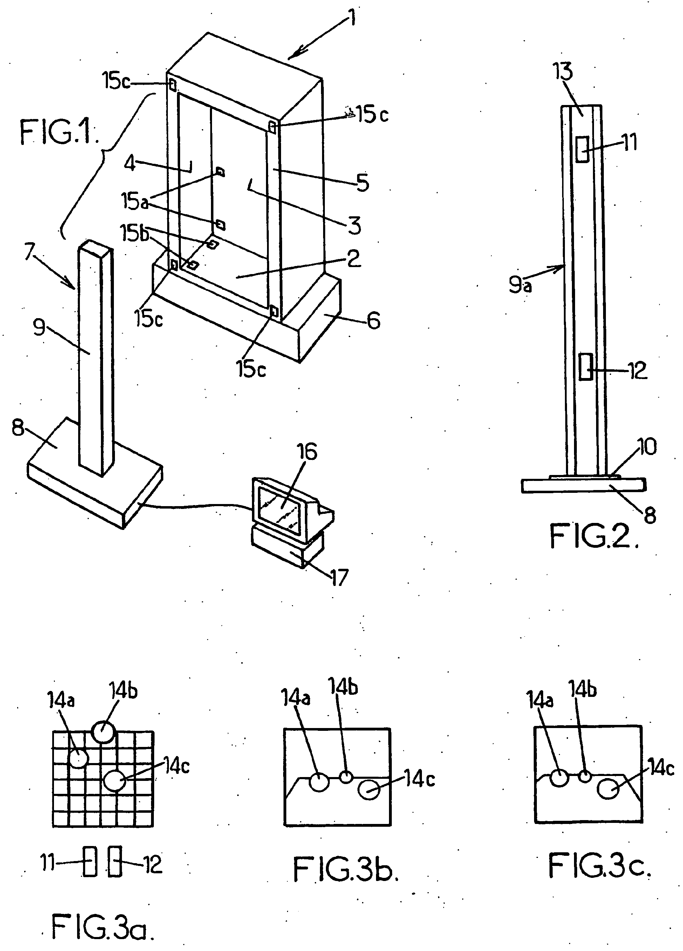

L'installation de métrologie représentée sur la figure 1 comporte une cabine 1 de taille suffisante pour contenir un sujet humain devant être soumis à la métrologie, de dimensions internes par exemple égales à 2m x 1m x 1m. La cabine 1 comporte un sol 2 et une paroi supérieure interne (masquée sur la figure 1) faisant face au sol 2, s'étendant sensiblement horizontalement. Elle comporte un fond 3 et une paire de parois latérales internes 4 opposées (dont une seule est visible sur la figure 1), par exemple de couleur uniforme bleue et s'étendant sensiblement verticalement entre le sol 2 et la paroi supérieure interne pour définir un espace de mesure interne entre ces parois.

La cabine 1 comporte également, de manière notable, une face avant 5 partiellement ouverte pour permettre l'entrée d'un sujet dans la cabine. La cabine 1 est éventuellement montée sur un socle 6 présentant des moyens de réglage (non représentés) permettant sa parfaite horizontalité.

L'installation comporte également un élément de visée et de prise de vue 7 réalisé dans le présent exemple sous la forme d'un pied 8 portant un bâti 9 tel qu'un montant vertical de hauteur sensiblement égale à la hauteur de la cabine, et qui sera décrit plus en détail par la suite, en relation avec la figure 2.

Le montant est disposé à quelques mètres de distance de la cabine en présentant une face avant 9a (non visible sur la figure 1) orientée en direction de la cabine 1. Latéralement, l'élément de visée et de prise de vue 7 peut être positionné sensiblement de façon centrale par rapport à la cabine 1. La position relative de l'élément de visée et de prise de vue 7 par rapport à la cabine peut être vérifiée lors de la mise en service de l'installation par un système d'alignement classique, par exemple du type comprenant une diode laser dans le pied 8 et un miroir d'auto-collimation correspondant à un endroit approprié du socle 6. Le réglage de la position relative des deux éléments peut être effectué précisément, par exemple à l'aide de moyens de réglage de la position horizontale du pied 8 du même type que ceux de la cabine 1, et par montage rotatif du montant 9 sur le pied 8 par l'intermédiaire d'une platine 10 (figure 2).

La face avant 9a du montant 9 est représentée sur la figure 2. Cette face avant fait face à la cabine 1, et comporte un premier dispositif de détection 11 apte à capter un rayonnement électromagnétique visible en provenance de la cabine, tel que par exemple un appareil photo numérique, ou une caméra numérique. Situé sous le premier dispositif de détection 11, on prévoit un deuxième dispositif 12, par exemple identique. On notera que les deux dispositifs de détection 11 et 12 prennent deux vues de la cabine selon des incidences différentes. Pour ce faire, les deux dispositifs de détection sont disposés de sorte que leurs axes focaux respectifs forment un angle non nul entre eux. Alternativement, les axes focaux de chaque dispositif pourraient être parallèles entre eux, et les appareils décalés l'un par rapport à l'autre le long du montant 9.

Le montant 9 porte en outre sur sa face avant 9a un organe de visée 13, qui peut être visé par le sujet prenant place dans la cabine 1. L'organe de visée est par exemple réalisé sous la forme d'une bande verticale sombre 13 s'étendant verticalement derrière les dispositifs de détection. Elle peut être de largeur réglable.

Bien que la description qui vient d'être faite cite deux appareils photos, on pourrait bien entendu utiliser d'avantage de dispositifs de détection présentant des incidences différentes deux à deux.

En outre, les dispositifs de détection 11, 12 et l'organe de visée ne sont pas nécessairement agencés comme décrit ci-dessus. L'agencement relatif qui vient d'être décrit pour les dispositifs de détection et l'organe de visée permet de prendre des images planes du sujet placé dans la cabine de face lorsqu'il regarde (lorsqu'il vise) l'organe de visée.

Les dispositifs de détection 11, 12 sont reliés à un système informatique 17 (figure 1) qui peut être adapté pour déclencher les prises de vue, pour stocker les images planes obtenues par les dispositifs de détection, et pour traiter ces images planes, par exemple à l'aide de la librairie Evision d'Euresys.

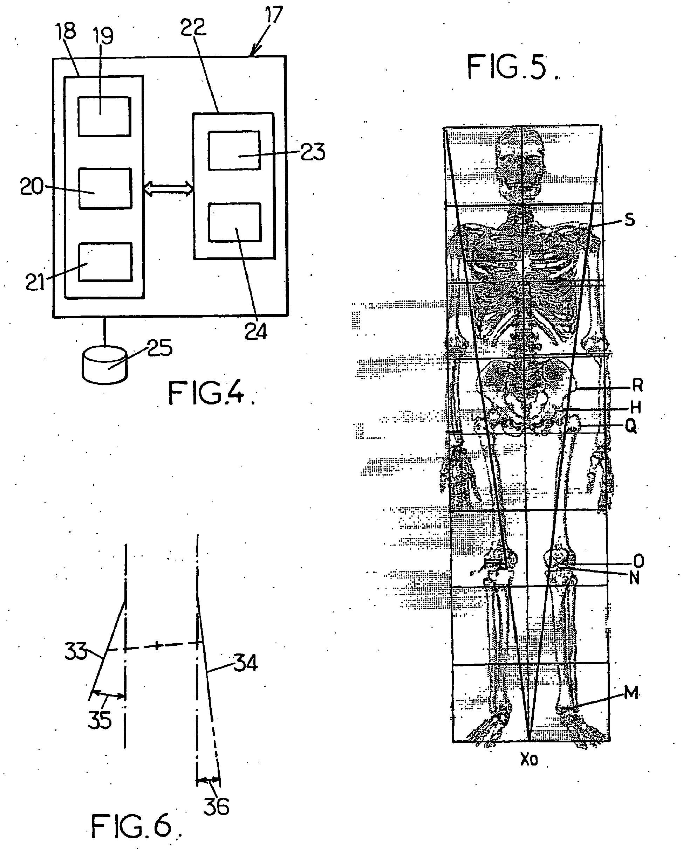

Le système informatique 17 est par exemple constitué comme représenté sur la figure 4. Il comporte une unité centrale 18 adaptée pour effectuer des calculs. A titre purement illustratif, l'unité centrale 18 comporte un système d'identification 19, un système d'appariement 20 et une unité de calcul 21 qui seront décrit plus en détails ci-après, et qui sont représentées ici comme différentes briques logicielles. Le système informatique comprend également une mémoire 22 comportant un modèle anatomique 23 et une relation 24 stockée suite à une étape de calibrage, et permettant de transformer des coordonnées bidimensionnelles d'un point anatomique du sujet sur chaque image plane en des coordonnées tridimensionnelles de ce point dans l'espace de mesure comme explicité plus en détail par la suite.

Le système informatique 17 peut également comporter ou être relié de manière distante à une base de données 25 contenant des données relatives à des sujets et à des examens antérieurs pour ces sujets.

Pour obtenir la relation, il est nécessaire de calibrer l'installation selon le principe de la stéréoscopie, qui sera rappelé ci-dessous en relation avec les figures 3a à 3c. Lors d'une mesure ultérieure sur un objet, les trois coordonnées dans l'espace de mesure de points quelconques peuvent par la suite être déterminées à partir de la géométrie du système, et de positions de la représentation de ces points sur chacune des images planes.

On notera à ce titre qu'il n'est pas nécessaire que le point anatomique dont on cherche les coordonnées tridimensionnelles possède une représentation visible sur chaque image plane, ce qui est rarement le cas en pratique. Il suffit que l'on puisse déterminer les coordonnées bidimensionnelles qu'aurait la représentation de ce point anatomique sur chaque image plane s'il y était visible. Ces coordonnées bidimensionnelles sont obtenues à l'aide du système d'identification 19 et du système d'appariement 20, comme illustré par la suite.

Le procédé employé permet la comparaison de deux images (celle de la Figure 3b prisé par le dispositif de détection 11 de la figure 3a, et celle de la Figure 3c prise par le dispositif de détection 12 de la figure 3a,) d'une même scène (comportant trois objets 14a, 14b, 14c) prises sous des points de vue différents dans le but d'obtenir des informations sur la profondeur des objets qui composent la scène.

Cette technique pourra être appliquée sur un volume de dimension isotrope mais la recherche de paires de points homologues (ou fusion stéréoscopique) sur les deux vues nécessaires à la détermination de la profondeur limite la densité en objets.

La relation entre la position tridimensionnelle d'un point P(x) dans le champ objet et sa représentation P(X) sur l'image plane fournie par chaque dispositif de détection 11, 12 est formalisée de la façon suivante :

$$X = F(x), \qquad\qquad (1)$$

où F est appelée fonction de "mapping". Cette fonction est généralement approchée par un polynôme de degré adapté aux phénomènes que l'on cherche à corriger :

$$F(x) = a_0 + a_1 x_1 + a_2 x_2 + a_3 x_3 + a_4 x_1^2 + a_5 x_1 x_2 + a_6 x_2^2 + a_7 x_1 x_3 + a_8 x_2 x_3 + a_9 x_3^2 + a_{10} x_1^3$$
$$+ a_{11} x_1^2 x_2 + a_{12} x_1 x_2^2 + a_{13} x_2^3 + a_{14} x_1^2 x_3 + a_{15} x_1 x_2 x_3 + a_{16} x_2^2 x_3 + a_{17} x_1 x_3^2 + a_{18} x_2 x_3^2 \qquad (2)$$

Par exemple, on pourra choisir une approche quadratique, où une approche de degré supérieur si la précision requise le nécessite.

Lors de la phase de calibration, on détermine tout d'abord un plan z=0 où la disparité (la différence de représentation entre les deux images planes) est nulle. La position des points est ensuite calculée par rapport à ce plan de référence. Puis, on cherche à obtenir des valeurs pour les paramètres $a_0$ à $a_9$ (dans le cas quadratique). Pour cela, lors de la calibration, on prend une première image plane de l'espace de mesure à l'aide du premier dispositif de détection 11, et une deuxième image plane de l'espace de mesure à l'aide du deuxième dispositif de détection 12.

Comme on a pu le constater en relation avec la figure 1, la cabine peut porter des cibles de calibration 15a, 15b, et 15c, de position et de taille connues par rapport à la cabine 1. Des cibles de calibration 15a sont disposées sur la face arrière 3 de la cabine 1, par exemple au nombre de 9 (en haut, en bas et au milieu sur les deux bords latéraux de la face arrière 3 et sur l'axe vertical médian de celle-ci). D'autres cibles de calibration 15c sont disposées sur la face avant 5 de la cabine, par exemple aux 4 coins. Enfin, des cibles de calibration 15b sont disposées sur le sol 2 de la cabine 1, par exemple en chaque coin.

En inversant l'équation (2) pour une pluralité de points connus de l'espace de mesure, on obtient la fonction de « mapping » pour l'espace de mesure.

Lors de la mise en service de l'installation, on peut par exemple procéder à sa calibration, qui dépend des positions relatives de l'élément de prise de vue et de visée 7 et de la cabine 1 dans la pièce dédiée. La calibration va par exemple être réalisée une fois pour toutes, et sera valable tant que les éléments constitutifs de l'installation ne seront pas déplacés. Si les appareils photos 11 et 12 sont montés mobiles (coulissants) sur la face avant 9a du dispositif de prise de vue et de visée, une calibration devra être effectuée pour chaque nouvelle position d'au moins un appareil photo.

On notera qu'on peut, de manière similaire, obtenir une fonction de « mapping » uniquement pour le sol 2, à partir des coordonnées et des tailles connues des cibles 15b disposées sur le sol. Elle permet de passer des coordonnées des représentations dans chaque image plane aux coordonnées du motif géométrique caractéristique dans un plan donné, en particulier le plan podal, correspondant au plan formé par le sol 2 de la cabine.

Le système informatique 17 est adapté pour traiter, par reconnaissance de formes, les images planes, pour en extraire l'information recherchée, comme décrit plus en détail par la suite. A ce titre, le système d'identification détecte sur une image plane de référence une région d'intérêt, qui contiendrait la représentation du point anatomique recherché si celui-ci était visible sur l'image plane. Puis, le système d'appariement 20 détermine sur chaque image plane les coordonnées bidimensionnelles qu'aurait le point anatomique, par recherche de corrélation entre la région d'intérêt définie sur l'image plane de référence et une deuxième image.

Le système d'identification peut utiliser un modèle anatomique 23 stocké dans la mémoire 22 du système informatique. Par exemple, on peut utiliser un modèle anatomique décrit en relation avec la figure 5, issue de la description d'anatomie artistique issue du manuel Struttura Uomo, publié par Neri Pozza editore. Selon ce modèle, une grille divise le corps en huit segments de même hauteur. Les huit segments correspondent à la taille du sujet. Ils sont divisés en deux colonnes en $X_0$ de sorte que chaque colonne comporte huit carrés. La numérotation des carrés commence par le bas pour se terminer en haut. L'axe Z est l'axe postéro-antérieur, l'axe Y est vertical, et X complète le triplet d'axes de sorte que (X, Y, Z) forme un repère orthogonal pour la cabine 1, d'origine $(X_0, Y_0, Z_0)$.

Pour la mesure des points de la moitié inférieure, l'alignement des colonnes depuis le plan du sol est conservé.

Pour la mesure des points de la moitié supérieure du corps les deux colonnes (limitées à quatre carrés) sont centrées sur l'oeil postural assimilé à l'oeil directeur en X'o (non représenté). Le décalage entre les deux verticales Xo et X'o est mesuré.

A partir de cette grille, les coordonnées bidimensionnelles de points anatomiques du modèle sont connus. Enfin, l'unité de calcul calcule la position tridimensionnelle du point anatomique dans l'espace de mesure, à partir des coordonnées relevées sur chaque image plane.

Le système qui vient d'être décrit fonctionne comme suit :

Un être humain se place dans la cabine, en vue de la mesure d'une caractéristique géométrique d'un segment anatomique. La caractéristique géométrique peut être une position, une longueur, ou une orientation d'un segment anatomique dans l'espace de mesure, ou par rapport à un autre segment anatomique. Cette mesure passe par l'évaluation des coordonnées tridimensionnelles dans l'espace de mesure d'au moins un point anatomique tel qu'une extrémité d'os, ou autre qui n'est pas nécessairement visible sur les images planes du sujet.

Le sujet peut se positionner dans toute position nécessaire à un examen, par exemple requise par un médecin. En particulier, celui-ci peut se positionner de manière la plus naturelle possible, en fixant « au loin » la ligne de visée 13 de l'élément de prise de vue et de visée 7. Alternativement, le sujet peut se positionner dans la position du modèle anatomique (figure 5). Dans les deux cas, les sujets observent une cible étroite en vision de loin. Ce calage par la perspective a pour but d'obtenir une reproductibilité des mesures, les sujets se replaçant spontanément aux mêmes endroits dans ces conditions.

Le système informatique 17 commande la prise d'une première image plane et d'une deuxième image plane, respectivement à l'aide des premier 11 et deuxième 12 dispositifs de détection, par exemple de manière simultanée (ou quasi-simultanée).

Chaque image plane comporte une représentation du segment anatomique. Les images planes peuvent par exemple être pré-traitées pour s'affranchir des conditions propres à l'environnement de travail (luminosité ambiante, etc...). Si les deux images sont prises par deux appareils 11, 12 ne présentant pas des axes focaux parallèles, elles peuvent être redressées pour présenter une configuration de stéréoscopie plane. Au moins l'une d'entre elles peut également être remise à l'échelle d'une image de référence, si nécessaire. Les images pré-traitées obtenues alors représentent exactement la situation présentée sur les figures 3a à 3c de deux images prises par deux appareils photos identiques décalés à axes focaux parallèle. Au moins une des images est recadrée en mettant en coïncidence un plan donné sur chaque image plane, par exemple le plan du fond 3 de la cabine, identifié par les cibles 15a.

Si deux représentations d'un même point du sujet ne sont pas exactement correspondantes sur les deux images planes obtenues après ce pré-traitement, c'est que ce point n'appartient pas au plan de fusion du fond de la cabine. Il présente une profondeur que l'on peut déterminer à partir des fonctions de « mapping » obtenus par le calibrage.

Il faut néanmoins identifier de manière rigoureuse les deux représentations d'un même point anatomique du sujet. Cela est a priori difficile car on ne dispose que d'une image de la peau du sujet. On peut par exemple procéder de la façon suivante : On commence par reconstruire sur une image plane de référence une grille correspondant à la grille du modèle anatomique. A cet effet, il est nécessaire de déterminer la position de l'origine de la grille, qui est par exemple choisie comme la position moyenne des deux pieds dans le plan podal. Par recherche de contrastes sur une image plane de référence, on détermine le contour de chaque pied. En se référant à la symétrie de l'enveloppe du pied, on détermine l'axe longitudinal de chaque pied sur l'image plane de référence. L'intersection de cet axe longitudinal et de l'enveloppe du pied précédemment déterminée permettent de déterminer les extrémités de chaque pied sur l'image plane de référence.

Pour chacun des quatre points correspondants aux deux extrémités de chacun des deux pieds déterminés sur l'image de référence, le système d'appariement détermine sur chaque autre image plane les coordonnées bidimensionnelles de ces points, comme cela sera explicité plus en détail par la suite. Puis, l'unité de calcul applique les fonctions de « mapping » aux points identifiés sur chaque image plane, pour obtenir les coordonnées tridimensionnelles des points anatomiques correspondants dans l'espace de mesure. Comme représenté sur la figure 6, on détermine pour chaque pied son milieu, et on détermine comme origine de la grille le point médian entre ces deux milieux.

La taille de la grille, qui, dans le modèle anatomique, correspond à un sujet de taille moyenne, est également modifiée pour s'adapter à la taille du sujet soumis à la mesure. Enfin, la grille est déformée pour être adaptée à l'incidence avec laquelle l'image plane a été prise. Sur la base de cette information sur l'origine, sur la taille du sujet, et sur l'incidence de la prise de l'image plane de référence, une grille est reconstruite pour le sujet.

La grille permet de définir des régions d'intérêt sur chaque image, les plus susceptibles de contenir le point anatomique recherché pour chaque segment sur l'image plane de référence.

Les paragraphes qui suivent contiennent une description de la manière dont les régions d'intérêt pour les différents points anatomiques considérés sont déterminées.

Pour les membres inférieurs, on peut procéder comme pour le pied pour détecter sur l'image plane de référence une région d'intérêt susceptible de contenir le point anatomique recherché pour chaque segment anatomique. Par recherche de contraste sur l'image de référence, le système d'identification identifie les contours du segment anatomique étudié. A partir du contour déterminé, l'axe longitudinal du segment anatomique est également déterminé. Puis, on définit une région d'intérêt correspondant à une position approximative du point anatomique à partir de la grille reconstruite, comme explicité dans le détail pour un exemple de réalisation ci-dessous.

L'axe longitudinal du pied 33, 34 est identifié en se référant à la symétrie de l'enveloppe du pied. L'angle d'ouverture de chaque pied 35, 36 sur le plan du sol, et l'angle d'ouverture global sont obtenus.

La position d'avancement d'un pied par rapport à l'autre dans le plan sagittal selon l'axe Z est identifiée.

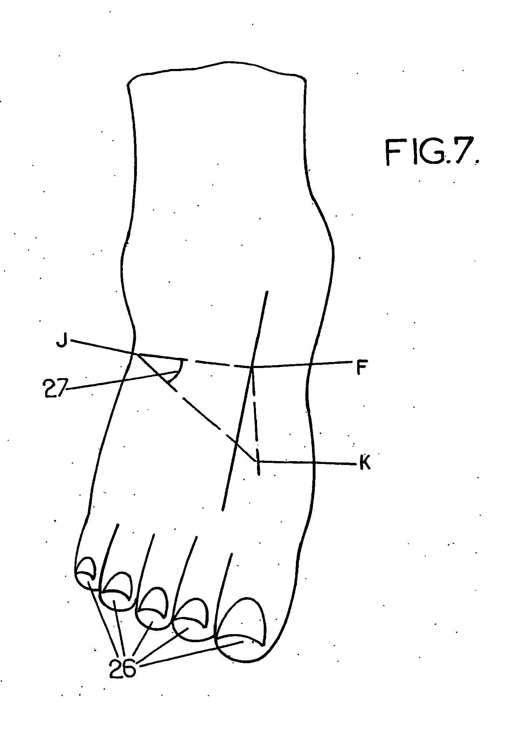

Le degré de varisation (supination) ou de valgisation (pronation) de chaque pied dans le plan frontal est identifié de la manière suivante en relation avec la figure 7 : Le point F est situé en regard de la projection de Yo (verticale de référence) sur le bord supérieur de l'axe de chaque pied, axe déterminé par le système d'identification comme décrit précédemment. Le point J est au contact du sol sur le bord externe perpendiculairement à l'axe du pied. Le point K est à l'aplomb du point F au niveau du sol. Le degré de varisation est déterminé manuellement par l'angle d'inclinaison 27 calculé entre les points F, J, K.

L'axe de chaque phalange 26 de chaque orteil par rapport à l'axe moyen du pied est identifié.

Pour identifier les chevilles dans le premier carré, on procède comme suit : Dans le plan frontal, l'identification porte sur les malléoles externes et internes qui sont les éléments anatomiques les plus proéminents identifiés au plus près du milieu horizontal du premier carré. Cette proéminence se détecte sur le contour interne et externe de la cheville. L'angle formé entre la projection de la droite issue de la localisation des points externes et internes et l'horizontale décrit l'angle bi-malléolaire dans le plan frontal. Le pointage manuel des malléoles renseigne la rotation des malléoles

La taille de chacune des jambes et l'axe de chaque jambe dans les plans frontaux et sagittaux sont identifiés de la manière suivante : La taille est calculée entre les point M et N. Le point M est situé dans le plan frontal au niveau inférieur de la jambe en son milieu en regard de la plus grande largeur de la cheville. Le point N est situé au niveau supérieur de la jambe au niveau de l'intersection des axes de la cuisse et de la jambe. Le degré d'inclinaison frontale est mesuré par rapport à la verticale. Le degré d'inclinaison sagittal est mesuré par rapport à la verticale, l'axe de la jambe correspond au bord antérieur de la jambe déterminé par le système d'identification. L'angle formé avec la cuisse indique l'existence d'une flexion ou flessum, ou d'une extension ou récurvatum.

La position des cuisses est identifiée de la manière suivante : Dans le plan frontal, la position des fémurs est obtenue en repérant les points O et Q. Le point O correspond au milieu de l'extrémité inférieure du fémur, il est déterminé comme étant au milieu du genou, au même niveau en Y que le point N. La distance pp' qui sépare le point O du bord externe de la cuisse est reportée en haut de la cuisse dans sa partie la plus externe et sert à déterminer la position du point Q. Le point Q se situe à la distance pp' de la partie externe la plus proéminente de la cuisse à la jonction du quatrième et du cinquième carré.

Le point Q est situé dans le plan sagittal, sur une verticale passant par $Y_o$. Le degré d'inclinaison frontale est mesuré par rapport à la verticale dans le plan frontal. Le degré d'inclinaison sagittal est mesuré par rapport à la verticale dans le plan sagittal.

Dans le plan frontal, la distance qui sépare le bord interne de chaque genou par rapport à la verticale est mesurée. Ces valeurs sont comparées. L'angle formé entre les jambes et les cuisses est mesuré. Les valeurs obtenues d'un coté par rapport à l'autre sont comparées. Dans le plan sagittal, l'angle formé entre les fémurs et les tibias permet d'identifier la présence de flexion ou d'extension.

La position de l'axe de la tête du fémur est repérée par le point H. Chez la femme, il se trouve dans le cinquième carré à 1/16 de la hauteur totale du sujet rapporté à l'axe vertical. Chez l'homme, la position de l'axe de la tête du fémur se trouve à 1/18 de la hauteur totale du sujet rapporté à l'axe vertical. Par rapport au bord inférieur, elle se trouve à ¼ de la longueur d'un carré. Dans le plan podal (X, Z), le point H est en moyenne situé en avant du point Q de 12°.

La position des épines iliaques antéro supérieures, qui reflète la position du bassin, est identifiée. Leur position se trouve en regard du point R. Dans le plan frontal, le point R est au croisement d'une ligne oblique de dedans en dehors de bas en haut partant de Xo, qui se termine dans l'angle supéro-externe du 8ème carreau et d'une ligne horizontale située aux 2/3 du bord inférieur du 5ème carré. La différence de positionnement en x des épines iliaques supérieures traduit la bascule et la translation du bassin. La différence de positionnement en Z des épines iliaques supérieures traduit la rotation du bassin.

Pour identifier des points abdominaux, il peut avoir été nécessaire de poser des moyens de marquage formant un contraste élevé avec la peau. Ces moyens de marquage peuvent par exemple être formés par de simples pastilles collées de manière amovible sur la peau aux endroits adaptés. Ces pastilles ne sont d'aucune gêne pour le sujet observé. Il est ensuite possible de reconstituer l'enveloppe corporelle.

L'articulation acromio-claviculaire est identifiée au point S. Le point S est situé dans le plan frontal dans le septième carré, sur la ligne oblique partant de Xo et se dirigeant dans le coin supéro-externe du huitième carré, au niveau de changement de contraste. Dans le plan sagittal le point S se trouve sur l'axe passant par Yo. Ces mesures renseignent la position des épaules.

Les pupilles sont identifiées automatiquement par recherche de motif dans les huitièmes carrés. Alternativement, on peut pointer manuellement les pupilles, en particulier pour les porteurs de verres correcteurs. Dans le plan frontal, la position relative de l'une par rapport à l'autre sert à évaluer l'horizontalité du regard. Nous identifions la positon des pupilles dans le plan sagittal. L'axe bi-pupillaire sert à situer la position de la tête dans le plan frontal, il permet de mesurer un écart à la verticale passant par $X_o$. Les conduits auditifs externes se trouvent dans le plan sagittal au point V. Le point V se trouve, dans le plan sagittal, sur l'axe passant par $Y_o$.

Une autre image plane peut être prise par chaque dispositif de détection après que le sujet s'est retourné dans la cabine.

Des points dorsaux peuvent être identifiés après que soient posés des repères au contact de la peau, comme pour les points abdominaux. L'ensemble des points a pour caractéristique de pouvoir être reliés les uns aux autres automatiquement. Une des applications est le dépistage et le suivi des scolioses.

Pour chaque point F à V identifié comme décrit précédemment, le système informatique définit en fait une région d'intérêt, par exemple de $15^2$ pixels autour du point identifié sur l'image plane de référence.

Du fait du décalage vertical des appareils photos, on sait que, sur une autre image plane, la représentation du même point se trouvera sur la même verticale que la représentation identifiée. Dans un espace de recherche dans la deuxième image plane, de hauteur H et de largeur L réglable par le système, on applique une fonction d'appariement aux deux images. Pour une pluralité de zones de $15^2$ pixels de l'espace de recherche, on calcule une corrélation entre la région d'intérêt identifiée sur l'image plane de référence et la zone en cours de la deuxième image.

La fonction de corrélation est susceptible de donner directement la zone de la deuxième image plane correspondant à la région d'intérêt de la première image plane.

Alternativement, on peut n'effectuer qu'un premier tri pour ne garder qu'un certain nombre de zones candidates de la deuxième image plane, pour lesquelles la fonction de corrélation donne un résultat satisfaisant.

Puis, on répète l'opération pour un certain nombre de zones (par exemple 4) situées en étoile autour de la région d'intérêt définie sur la première image plane. A l'issu des résultats obtenus pour ces 5 zones, on définit à coup sûr la zone de la deuxième image plane correspondant à la région d'intérêt définie sur la première image plane.

On peut prévoir de stocker dans le système informatique les positions des représentations sur chaque image plane.

A partir de la relation obtenue lors de la calibration de l'installation, et des coordonnées bidimensionnelles des points anatomiques sur chaque image plane, on calcule la position tridimensionnelle exacte du point anatomique.

Cette position tridimensionnelle peut en elle-même constituer l'information géométrique recherchée. Alternativement, en procédant de même pour un deuxième point anatomique du segment, on peut obtenir la position géométrique tridimensionnelle d'un deuxième point anatomique du sujet, et de là, une longueur caractéristique, ou une orientation caractéristique du segment anatomique.

Alternativement, en procédant de même pour un deuxième objet, on peut obtenir comme information géométrique un rapport de longueur entre deux segments, ou une orientation relative de deux segments.

On notera en particulier que si on ne cherche qu'à obtenir des grandeurs relatives entre segments, ou des angulations, on n'a pas forcément besoin de rapporter les mesures obtenues en grandeurs réelles, et on peut travailler dans une échelle relative (par exemple en unités « largeur de cabine »).

Ce sont ces positions tridimensionnelles qui sont utilisées pour déterminer les longueurs et les angulations décrites ci-dessus pour les points F à v.

Pour identifier plusieurs motifs repérés chacun par une pastille de forme donnée, on peut commencer par traiter l'image de référence pour que le système informatique applique une fonction de recherche de motifs à l'image plane de référence, pour retrouver un motif donnée dans l'image plane de référence. Pour chaque motif identifié, le traitement qui vient d'être décrit est, par la suite, appliqué.

Le système informatique peut ainsi retrouver automatiquement l'ensemble des marques posées sur le corps du sujet.

Toutes les mesures automatiques peuvent être réalisées manuellement. Tout point sélectionné manuellement peut être localisé et comparé aux mesures automatiques.

La ligne verticale passant par Xo est théoriquement médiane de segment en segment. Tout décalage dans l'alignement d'un segment au segment sus jacent est quantifié.

Ainsi, les mesures obtenues pour le sujet peuvent être comparées avec le modèle anatomique standard qui vient d'être décrit.

Alternativement, ou ensuite, d'autres mesures peuvent être effectuées dans une position « naturelle » du sujet, ne correspondant pas à une position anatomique précise définie par un manuel d'anatomie. De telles mesures peuvent en particulier être utiles pour renseigner sur certaines angulations relatives de membres, ou pour suivre l'évolution de la posture d'un patient au cours du temps lors de visites périodiques.

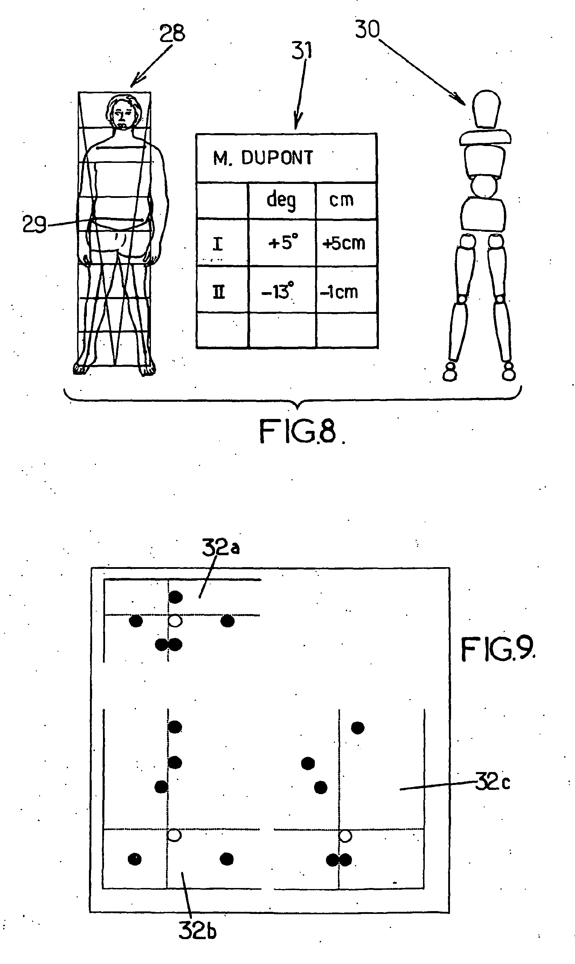

Le résultat des mesures peut être présenté sur l'écran 16 du système informatique 17 comme représenté sur la figure 8.

Sur celle-ci, la fenêtre de gauche 28 affiche une des images planes du sujet prise par l'un des dispositifs de détection 11 ou 12. La grille reconstruite est également affichée. Sur cette image, des coordonnées des points anatomiques ont également été obtenues (voir par exemple 29), et les positions tridimensionelles correspondantes ont pu être reconstruites, et sont affichées en superposition sur la photo ou sous la forme d'un modèle (fenêtre de droite 30). Une fenêtre centrale 31 peut représenter les résultats numériques de certaines mesures effectuées. De même, comme représenté sur la figure 9, les positions géométriques relatives des vertèbres peuvent être affichées en vue de dessus, de face, ou de côté, sur trois fenêtres distinctes 32a, 32b, 32c, respectivement.

Pour le suivi des sujets, les données obtenues peuvent être, avec l'accord du sujet, stockées de manière confidentielle dans une base de données 25 accessible depuis le système informatique 13. Celle-ci peut comprendre, pour chaque sujet, des informations statiques (Nom, Prénom, N° d'identification, Date de naissance ou âge, sexe, caractéristique génétique, etc...), des informations dynamiques par consultation (Date, Evaluation visuelle analogique, données mesurées, informations spécifiques à la consultation, etc...), et des données relatives aux mesures effectuées (paramètres de calibration, configuration des dispositifs de prise d'image, configuration spatiale du système, etc...).

Finalement, des déplacements de zones anatomiques peuvent être mesurés en reproduisant le système ci-dessus au moyen de prise d'images rapides pendant le mouvement du sujet dans la cabine 1.

Les mesures peuvent être effectuées librement dans une position imposée pour comparaison avec un modèle anatomique, en position de repos, en position d'inclinaison maximale, voire dans toute position jugée utile.

L'installation décrite ci-dessus peut également être couplée à une platine de stabilométrie, de baropodométrie, ou autre, permettant d'évaluer en outre les appuis au sol d'un individu. Un tel dispositif se présente comme une plateforme à capteurs de pression, supportant les pieds de l'individu. La mesure en pression permet de confirmer et/ou compléter la détection d'une dissymétrie dans la posture de l'individu, par corrélation avec les mesures obtenues par stéréoscopie au sens de l'invention. Une telle plateforme est couramment commercialisée notamment sous la référence AM3CUBE®.

## Revendications

**1.** Installation de mesure d'au moins une caractéristique géométrique d'un segment anatomique, ladite caractéristique géométrique étant associée à au moins un point anatomique d'un sujet placé dans un espace de mesure, le point anatomique du sujet présentant des coordonnées tridimensionnelles dans l'espace de mesure, à partir d'une pluralité d'images planes de l'espace de mesure prises chacune par un dispositif de détection (11, 12) adapté pour détecter un rayonnement électromagnétique visible provenant de l'espace de mesure, chaque image plane comprenant une représentation dudit segment anatomique, lesdites images planes étant distinctes deux à deux, ladite installation comprenant un système informatique de mesure (17) comprenant :

- un système d'identification (19), adapté pour identifier sur une image plane une région d'intérêt contenant ledit point anatomique du sujet,
- un système d'appariement (20), adapté pour déterminer sur chaque image plane, des coordonnées bidimensionnelles d'une représentation du point anatomique, par recherche de corrélation entre la région d'intérêt et une zone correspondante sur chaque autre image,
- une relation (24) entre une mesure géométrique sur chaque image plane et une grandeur dans l'espace de mesure, ladite relation ayant été préalablement établie lors d'une étape de calibrage de l'installation de mesure,
- une unité de calcul (21) adaptée pour déterminer les coordonnées tridimensionnelles dudit point anatomique du sujet dans l'espace de mesure sur la base desdites coordonnées bidimensionnelles déterminées par le système d'appariement, et de ladite relation,
**caractérisé en ce que**
l'installation de mesure comprend en outre un modèle anatomique (23) du segment, ledit modèle anatomique comprenant au moins un point anatomique modèle correspondant au point anatomique du sujet, ledit système d'identification étant adapté pour identifier sur une image plane ladite région d'intérêt à partir dudit modèle anatomique et d'un traitement de recherche de contraste sur ladite image plane.

**2.** Installation de mesure selon la revendication 1, comprenant en outre une pluralité de dispositifs de détection (11, 12) d'un rayonnement électromagnétique visible provenant de l'espace de mesure, chacun adapté pour prendre une image plane de l'espace de mesure, chaque image plane comprenant une représentation dudit segment anatomique, lesdites images planes étant distinctes deux à deux.

**3.** Installation de mesure selon la revendication 2, dans laquelle lesdits dispositifs de détection (11, 12) sont disposés chacun pour prendre une image plane de l'espace de mesure selon une incidence, lesdites incidences, associées chacune à un dispositif de détection, étant distinctes deux à deux.

**4.** Installation de mesure selon l'une des revendications précédentes, dans laquelle le système d'appariement (20) est adapté pour détecter une première zone sur une première image plane dans ladite région d'intérêt, et pour traiter au moins une autre image plane pour reconnaître une zone similaire à ladite première zone sur chaque autre image plane.

**5.** Installation de mesure selon l'une quelconque des revendications précédentes, dans laquelle, le segment anatomique comprend au moins deux points anatomiques de sujet, ledit modèle anatomique (23) comprenant au moins un point anatomique modèle correspondant à chaque point anatomique du sujet,
ladite unité de calcul (21) étant adaptée pour déterminer ladite caractéristique géométrique à partir des coordonnées tridimensionnelles de chaque point anatomique dans l'espace de mesure.

6.  Installation de mesure selon l'une des revendications précédentes, dans laquelle ladite caractéristique géométrique est choisie dans le groupe consistant en une position du segment anatomique dans l'espace de mesure, une longueur du segment anatomique, une orientation du segment anatomique par rapport à une droite de l'espace de mesure, une orientation du segment anatomique par rapport à un plan de l'espace de mesure, une orientation du segment anatomique par rapport à un autre segment anatomique de l'espace de mesure.

7.  Installation de mesure selon l'une quelconque des revendications précédentes, comprenant en outre un système de visée (13) disposé pour être visible du sujet quand il est dans l'espace de mesure.

8.  Installation de mesure selon l'une quelconque des revendications précédentes, comprenant un système de calibration (11, 12, 15a, 15b, 15c) de l'espace de mesure adapté pour fournir ladite relation, et comprenant au moins une cible (15a, 15b, 15c) dont au moins une caractéristique géométrique dans l'espace de mesure est connue,
ladite cible présentant une représentation sur une image plane prise par chaque dispositif de détection en l'absence du sujet dans l'espace de mesure,
l'unité de calcul étant adaptée pour déterminer ladite relation à partir de ladite caractéristique géométrique de la cible (15a, 15b, 15c) dans l'espace de mesure, et d'une propriété géométrique de ladite représentation sur chaque image plane.

9.  Installation de mesure selon la revendication 8 comprenant une cabine (1) délimitant l'espace de mesure, ladite cabine (1) portant lesdites cibles (15a, 15b, 15c).

10. Installation de mesure selon l'une des revendications précédentes, comprenant en outre au moins une pastille adaptée pour être fixée de manière amovible sur le segment anatomique,
ledit système d'identification étant adapté pour identifier, sur au moins une image plane, une représentation de ladite pastille en tant que région d'intérêt.

11. Procédé de mesure d'au moins une caractéristique géométrique d'un segment anatomique,
ladite caractéristique géométrique étant associée à au moins un point anatomique d'un sujet placé dans un espace de mesure, le point anatomique du sujet présentant des coordonnées tridimensionnelles dans l'espace de mesure,
à partir d'une pluralité d'images planes de l'espace de mesure prises chacune par un dispositif de détection (11, 12) adapté pour détecter un rayonnement électromagnétique visible provenant de l'espace de mesure, chaque image plane comprenant une représentation dudit segment anatomique,
lesdites images planes étant distinctes deux à deux,
ledit procédé comprenant les étapes suivantes :

(a) on identifie sur une image plane une région d'intérêt contenant ledit point anatomique de sujet,
(b) on détermine sur chaque image plane des coordonnées bidimensionnelles d'une représentation du point anatomique du sujet, par recherche de corrélation entre la région d'intérêt et une zone correspondante sur chaque autre image,
(c) on détermine les coordonnées tridimensionnelles dudit point anatomique du sujet dans l'espace de mesure sur la base desdites coordonnées bidimensionnelles déterminées par le système d'appariement, et d'une relation entre une mesure géométrique sur chaque image plane et une grandeur dans l'espace de mesure, ladite relation ayant été préalablement établie lors d'une étape de calibrage de l'installation de mesure,
**caractérisé en ce que**
la région d'intérêt est identifiée à partir d'un traitement de recherche de contraste sur ladite image plane, et d'un modèle anatomique (23) du segment, ledit modèle anatomique comprenant au moins un point anatomique modèle correspondant au point anatomique du sujet.

12. Procédé de mesure selon la revendication 11 dans lequel ledit modèle anatomique (23) comprend une pluralité de points anatomiques modèles correspondant à des points anatomiques du sujet, et dans lequel on met en oeuvre pour chaque point anatomique du sujet les étapes (a), (b) et (c).

13. Procédé de mesure selon l'une des revendications 11 et 12, comprenant en outre une étape de calibration au cours de laquelle on définit une relation (24) entre une mesure géométrique sur chaque image plane et une grandeur dans l'espace de mesure.

**14.** Programme d'ordinateur comprenant des codes de programme pour la mise en oeuvre d'un procédé de mesure selon l'une des revendications 11 à 13 lorsqu'il est exécuté sur une machine programmable (17).

**Claims**

**1.** An installation for measuring at least one geometrical characteristic of an anatomical segment,
said geometrical characteristic being associated with at least one anatomical point of an individual presenting three-dimensional coordinates in the measurement space, by using a plurality of plane images of the measurement space, each taken by a detector device (11, 12) adapted to detect visible electromagnetic radiation coming from the measurement space, each plane image including a representation of said anatomical segment,
said plane images being distinct in pairs,
said installation comprising a measurement computer system (17) comprising:

- an identification system (19) suitable for identifying on a plane image a region of interest containing said anatomical point of the individual;
- a pairing system (20) adapted to determine on each plane image, two-dimensional coordinates of a representation of the anatomical point by searching for correlation between the region of interest and a corresponding zone on each other image;
- a relationship (24) between a geometrical measurement on each plane image and a magnitude in the measurement space, said relationship being previously established during a step of calibrating the measurement installation;
- a calculation unit (21) adapted to determine the three-dimensional coordinates of said anatomical point of the individual in the measurement space on the basis of said two-dimensional coordinates determined by the pairing system, and of said relationship,
**characterized in that**
the measurement installation further comprises an anatomical model (23) of the segment, said anatomical model comprising at least one model anatomical point corresponding to the anatomical point of the individual, said identification system being adapted to identify on a plane image said region of interest by using said anatomical model and processing by searching for contrast in said plane image.

**2.** A measurement installation according to claim 1, further comprising a plurality of detector devices (11, 12) for detecting visible electromagnetic radiation coming from the measurement space, each being adapted to take a plane image of the measurement space, each plane image including a representation of said anatomical segment, said plane images being distinct in pairs.

**3.** A measurement installation according to claim 2, in which said detector devices (11, 12) are each disposed to take a plane image of the measurement space at an angle of incidence,
said angles of incidence, each associated with a detector device, being distinct in pairs.

**4.** A measurement installation according to any preceding claim, in which the pairing system (20) is adapted to detect a first zone on a first plane image in said region of interest, and to process at least one other plane image in order to recognize a zone similar to said first zone in each other plane image.

**5.** A measurement installation according to any preceding claim, in which, the anatomical segment includes at least two anatomical points of the individual,
said anatomical model (23) comprising at least one model anatomical point corresponding to each anatomical point of the individual,
said calculation unit (21) being adapted to determine said geometrical characteristic on the basis of the three-dimensional coordinates of each anatomical point in the measurement space.

**6.** A measurement installation according to any preceding claim, in which said geometrical characteristic is selected from the group consisting in: a position of the anatomical segment in the measurement space; a length of the anatomical segment; an orientation of the anatomical segment relative to a straight line in the measurement space; an orientation of the anatomical segment relative to a plane in the measurement space; and an orientation of the anatomical segment relative to another anatomical segment in the measurement space.

7. A measurement installation according to any preceding claim, further comprising a sighting system (13) disposed to be visible to the individual when in the measurement space.

8. A measurement installation according to any preceding claim, including a system (11, 12, 15a, 15b, 15c) for calibrating the measurement space adapted to supply said relationship, and comprising at least one target (15a, 15b, 15c) having at least one geometrical characteristic in the measurement space that is known,
said target presenting a representation on a plane image taken by each detector device in the absence of the individual in the measurement space; and
the calculation unit being adapted to determine said relationship on the basis of said geometrical characteristic of the target (15a, 15b, 15c) in the measurement space, and of a geometrical property of said representation on each plane image.

9. A measurement installation according to claim 8, including a cabin (1) defining the measurement space, said cabin (1) carrying said targets (15a, 15b, 15c).

10. A measurement installation according to any preceding claim, further comprising at least one sticker adapted to be secured releasably on the anatomical segment,
said identification system being adapted to identify on at least one plane image, a representation of said sticker at a region of interest.

11. A method of measuring at least one geometrical characteristic of an anatomical segment,
said geometrical characteristic being representative of the posture of an individual placed in a measurement space,
said geometrical characteristic being further associated with at least one anatomical point of the individual presenting three-dimensional coordinates in the measurement space, on the basis of a plurality of plane images of the measurement space, each taken by a detector device (11, 12) adapted to detect visible electromagnetic radiation coming from the measurement space, each plane image including a representation of said anatomical segment,
said plane images being distinct in pairs,
said method comprising the following steps:

a) identifying on a plane image a region of interest containing said anatomical point of the individual;
b) determining on each plane image two-dimensional coordinates of a representation of the anatomical point of the individual by searching for correlation between the region of interest and a corresponding zone on each other image; and
c) determining the three-dimensional coordinates of said anatomical point of the individual in the measurement space on the basis of said two-dimensional coordinates determined by the pairing system, and of a relationship between a geometrical measurement on each plane image and a magnitude in the measurement space, said relationship being established previously during a step of calibrating the measurement installation,
**characterized in that**
the region of interest is identified from a process of searching for contrast on said plane image, and an anatomical model (23) of the segment, said anatomical model including at least one model anatomical point corresponding to the anatomical point of the individual.

12. A measurement method according to claim 11, in which said anatomical model (23) includes a plurality of model anatomical points corresponding to anatomical points of the individual, and in which steps a), b), and c) are implemented for each anatomical point of the individual.

13. A measurement method according to any one of claims 11 or 12, further comprising a calibration step during which a relationship (24) is defined between a geometrical measurement on each plane image and a magnitude in the measurement space.

14. A computer program including program code for implementing a measurement method according to any one of claims 11 to 13 on being executed by a programmable machine (17).

**Patentansprüche**

1. Anlage zur Messung mindestens eines geometrischen Merkmals eines anatomischen Segments,
wobei das geometrische Merkmal mindestens einem anatomischen Punkt einer Person zugeordnet ist, die sich in einem Messraum befindet, wobei der anatomische Punkt der Person dreidimensionale Koordinaten im Messraum hat, ausgehend von mehreren ebenen Bildern des Messraums, die je von einer Erfassungsvorrichtung (11, 12) aufgenommen werden, die geeignet ist, um eine vom Messraum kommende, sichtbare elektromagnetische Strahlung zu erfassen, wobei jedes ebene Bild eine Darstellung des anatomischen Segments enthält,

wobei die ebenen Bilder paarweise verschieden sind,

wobei die Anlage ein EDV-Messsystem (17) enthält, das enthält:

- ein Identifikationssystem (19), das geeignet ist, um in einem ebenen Bild einen zweckdienlichen Bereich zu identifizieren, der den anatomischen Punkt der Person enthält,
- ein Paarbildungssystem (20), das geeignet ist, um in jedem ebenen Bild zweidimensionale Koordinaten einer Darstellung des anatomischen Punkts durch Korrelationssuche zwischen dem zweckdienlichen Bereich und einer entsprechenden Zone in jedem anderen Bild zu bestimmen,
- eine Beziehung (24) zwischen einer geometrischen Messung in jedem ebenen Bild und einer Größe im Messraum, wobei die Beziehung vorher in einem Kalibrierschritt der Messanlage erstellt wurde,
- eine Recheneinheit (21), die geeignet ist, um die dreidimensionalen Koordinaten des anatomischen Punkts der Person im Messraum auf der Basis der vom Paarbildungssystem bestimmten zweidimensionalen Koordinaten und der Beziehung zu bestimmen,

**dadurch gekennzeichnet, dass**

die Messanlage außerdem ein anatomisches Modell (23) des Segments enthält, wobei das anatomische Modell mindestens einen anatomischen Modellpunkt enthält, der dem anatomischen Punkt der Person entspricht, wobei das Identifikationssystem geeignet ist, um in einem ebenen Bild den zweckdienlichen Bereich ausgehend von dem anatomischen Modell und von einer Verarbeitung der Kontrastsuche in dem ebenen Bild zu identifizieren.

2. Messanlage nach Anspruch 1, die außerdem mehrere Erfassungsvorrichtungen (11, 12) einer vom Messraum kommenden, sichtbaren elektromagnetischen Strahlung enthält, die je geeignet sind, um ein ebenes Bild des Messraums aufzunehmen, wobei jedes ebene Bild eine Darstellung des anatomischen Segments enthält, wobei die ebenen Bilder paarweise verschieden sind.

3. Messanlage nach Anspruch 2, bei der die Erfassungsvorrichtungen (11, 12) je angeordnet sind, um ein ebenes Bild des Messraums gemäß einem Einfallwinkel aufzunehmen, wobei die Einfallwinkel, die je einer Erfassungsvorrichtung zugeordnet sind, paarweise verschieden sind.

4. Messanlage nach einem der vorhergehenden Ansprüche, bei der das Paarbildungssystem (20) geeignet ist, um eine erste Zone in einem ersten ebenen Bild in der zweckdienlichen Zone zu erfassen, und um mindestens ein weiteres ebenes Bild zu verarbeiten, um eine Zone ähnlich der ersten Zone in jedem anderen ebenen Bild zu erkennen.

5. Messanlage nach einem der vorhergehenden Ansprüche, bei der das anatomische Segment mindestens zwei anatomische Punkte der Person enthält,

wobei das anatomische Modell (23) mindestens einen anatomischen Modellpunkt enthält, der jedem anatomischen Punkt der Person entspricht,

wobei die Recheneinheit (21) geeignet ist, um das geometrische Merkmal ausgehend von den dreidimensionalen Koordinaten jedes anatomischen Punkts im Messraum zu bestimmen.

6. Messanlage nach einem der vorhergehenden Ansprüche, bei der das geometrische Merkmal aus der Gruppe ausgewählt wird, die aus einer Position des anatomischen Segments im Messraum, einer Länge des anatomischen Segments, einer Ausrichtung des anatomischen Segments bezüglich einer Geraden des Messraums, einer Ausrichtung des anatomischen Segments bezüglich einer Ebene des Messraums, einer Ausrichtung des anatomischen Segments bezüglich eines anderen anatomischen Segments des Messraums besteht.

7. Messanlage nach einem der vorhergehenden Ansprüche, die außerdem ein Visiersystem (13) enthält, das angeordnet ist, um für die Person sichtbar zu sein, wenn sie im Messraum ist.

8. Messanlage nach einem der vorhergehenden Ansprüche, die ein Kalibriersystem (11, 12, 15a, 15b, 15c) des Messraums enthält, das geeignet ist, um die Beziehung zu liefern, und mindestens ein Ziel (15a, 15b, 15c) enthält, von dem mindestens ein geometrisches Merkmal im Messraum bekannt ist,

wobei das Ziel eine Darstellung in einem ebenen Bild aufweist, das von jeder Erfassungsvorrichtung in Abwesenheit der Person im Messraum aufgenommen wird,
wobei die Recheneinheit geeignet ist, um die Beziehung ausgehend von dem geometrischen Merkmal des Ziels (15a, 15b, 15c) im Messraum und von einer geometrischen Eigenschaft der Darstellung in jedem ebenen Bild zu bestimmen.

9. Messanlage nach Anspruch 8, die eine den Messraum begrenzende Kabine (1) enthält, wobei die Kabine (1) die Ziele (15a, 15b, 15c) trägt.

10. Messanlage nach einem der vorhergehenden Ansprüche, die außerdem mindestens ein Plättchen enthält, das geeignet ist, um lösbar auf dem anatomischen Segment befestigt zu werden,
wobei das Identifikationssystem geeignet ist, um in mindestens einem ebenen Bild eine Darstellung des Plättchens als zweckdienlicher Bereich zu identifizieren.

11. Verfahren zur Messung mindestens eines geometrischen Merkmals eines anatomischen Segments,
wobei das geometrische Merkmal mindestens einem anatomischen Punkt einer in einem Messraum befindlichen Person entspricht, wobei der anatomische Punkt der Person dreidimensionale Koordinaten im Messraum aufweist, ausgehend von mehreren ebenen Bildern des Messraums, die je von einer Erfassungsvorrichtung (11, 12) aufgenommen werden, die geeignet ist, um eine vom Messraum kommende, sichtbare elektromagnetische Strahlung zu erfassen, wobei jedes ebene Bild eine Darstellung des anatomischen Segments enthält,
wobei die ebenen Bilder paarweise verschieden sind,
wobei das Verfahren die folgenden Schritte enthält:

(a) in einem ebenen Bild wird ein zweckdienlicher Bereich identifiziert, der den anatomischen Punkt der Person enthält,
(b) in jedem ebenen Bild werden zweidimensionale Koordinaten einer Darstellung des anatomischen Punkts der Person durch Korrelationssuche zwischen dem zweckdienlichen Bereich und einer entsprechenden Zone in jedem anderen Bild bestimmt,
(c) die dreidimensionalen Koordinaten des anatomischen Punkts der Person im Messraum werden auf der Basis der vom Paarbildungssystem bestimmten zweidimensionalen Koordinaten und von einer Beziehung zwischen einer geometrischen Messung in jedem ebenen Bild und einer Größe im Messraum bestimmt, wobei die Beziehung vorher in einem Schritt der Kalibrierung der Messanlage erstellt wurde,
**dadurch gekennzeichnet, dass**
der zweckdienliche Bereich ausgehend von einer Kontrastsuche-Verarbeitung im ebenen Bild und von einem anatomischen Modell (23) des Segments identifiziert wird, wobei das anatomische Modell mindestens einen anatomischen Modellpunkt enthält, der dem anatomischen Punkt der Person entspricht.

12. Messverfahren nach Anspruch 11, bei dem das anatomische Modell (23) mehrere anatomische Modellpunkte enthält, die anatomischen Punkten der Person entsprechen, und bei dem für jeden anatomischen Punkt der Person die Schritte (a), (b) und (c) durchgeführt werden.

13. Messverfahren nach einem der Ansprüche 11 und 12, das außerdem einen Kalibrierungsschritt enthält, während dem eine Beziehung (24) zwischen einer geometrischen Messung in jedem ebenen Bild und einer Größe im Messraum definiert wird.

14. Computerprogramm, das Programmcodes zur Durchführung eines Messverfahrens nach einem der Ansprüche 11 bis 13 enthält, wenn es in einer programmierbaren Maschine (17) ausgeführt wird.

FIG.1.

FIG.2.

FIG.3a.

FIG.3b.

FIG.3c.

FIG.4.

FIG.5.

FIG.6.

FIG.7.

FIG.8.

FIG.9.

**EP 1 853 164 B1**

## RÉFÉRENCES CITÉES DANS LA DESCRIPTION

**Documents brevets cités dans la description**

- DE 4216458
- US 5016173 A
- US 2004032595 A
- US 2004228517 A
- US 5457325 A
- US 2001030754 A